# EUROPÄISCHE PATENTSCHRIFT

(11) **EP 2 248 538 B1**
(45) Veröffentlichungstag und Bekanntmachung des Hinweises auf die Patenterteilung: **16.03.2016**
(21) Anmeldenummer: 10004917.0
(22) Anmeldetag: 10.05.2010
(51) Int. Cl.: A61L 2/10, B23Q 11/00, C02F 1/32

(54) **Vorrichtung und Verfahren zur UV-Behandlung von Flüssigkeiten**
Method and device for UV processing liquids
Dispositif et procédé destinés au traitement avec UV des liquides

(30) Priorität: 09.05.2009 DE 102009020687; 19.06.2009 DE 102009029911
(43) Veröffentlichungstag der Anmeldung: 10.11.2010
(73) Patentinhaber: Kaushal, Christian, 47053 Duisburg (DE)
(72) Erfinder: Kaushal, Christian, 47053 Duisburg (DE)
(74) Vertreter: Ring & Weisbrodt

(56) Entgegenhaltungen:
- EP-A1- 1 702 678
- WO-A1-95/06007
- DE-C- 251 320
- US-A- 5 433 866

## Beschreibung

Die vorliegende Erfindung betrifft eine Vorrichtung zur Behandlung von flüssigen Medien, insbesondere von Kühl- und Schmierstoffen, aufweisend wenigstens eine Zuführung für das zu behandelnde flüssige Medium, ein dreidimensionales Objekt, auf welches das flüssige Medium aufbringbar ist, wenigstens eine ultraviolette Strahlungsquelle zur Bestrahlung eines aus dem flüssigen Medium erzeugten Medienfilms, wobei die Wellenlänge der ultravioletten Strahlung und die Dicke des Medienfilms derart aufeinander abgestimmt sind, dass der Medienfilm im Wesentlichen vollständig von der ultravioletten Strahlung durchdringbar ist, und eine Auffangeinrichtung für das behandelte flüssige Medium zur nachfolgenden Nutzung des behandelten Mediums.

Des Weiteren betrifft die vorliegende Erfindung ein Verfahren zur Behandlung von flüssigen Medien, insbesondere von Kühl- und Schmierstoffen, wobei aus dem flüssigen Medium nach Aufbringung auf ein dreidimensionales Objekt ein Medienfilm erzeugt wird, welcher unter Einwirkung von ultravioletter Strahlung sterilisiert wird.

Bei mechanischen Bearbeitungsverfahren mittels Werkzeugmaschinen werden in der Fertigungstechnik regelmäßig Kühlschmiermittel oder Kühlschmierstoffe eingesetzt. Diese Kühlschmiermittel beziehungsweise Kühlschmierstoffe sollen insbesondere die bei der Bearbeitung von Werkstücken entstehende Wärme abführen und durch Schmierung die bei der Bearbeitung auftretende Reibung zwischen Werkzeug und Werkstück reduzieren. Ferner werden die Kühlschmiermittel beziehungsweise die Kühlschmierstoffe bei zerspanender Bearbeitung von Werkstücken zum Abtransport der bei der Bearbeitung entstehenden Späne genutzt.

Als Kühlschmiermittel werden üblicherweise flüssige Medien, wie Wasser oder Öle, eingesetzt, welchen diverse Zusatzstoffe zugesetzt werden. Aus Kostengründen wird das Kühlschmiermittel nach Gebrauch üblicherweise aufgefangen, um es erneut einsetzen zu können. Hierzu werden die Kühlschmiermittel regelmäßig einer mechanischen Reinigung unterzogen. Problematisch ist, dass sich insbesondere in Kühlschmiermitteln auf Wasserbasis Keime, wie Bakterien und/oder Pilze, bilden können. Diese Keime können Zersetzungsprozesse des Kühlschmiermittels zur Folge haben, wodurch die Wirksamkeit des Kühlschmiermittels herabgesetzt wird. Zudem kann ein Keimbefall des Kühlschmiermittels zu technischen Problemen führen, beispielsweise weil es durch den Befall zu Ablagerungen in den Zuführungen, welche das Kühlschmiermittel dem Bearbeitungsbereich zuführen, kommen kann, wodurch eine hinreichende Versorgung mit Kühlschmiermittel mitunter nicht mehr gegeben ist. Darüber hinaus können Keime in Kühlschmiermitteln auch zu gesundheitlichen Problemen führen. Da der Einsatz von chemischen Zusatzstoffen zur Verhinderung von Keimbildung ebenfalls technische Probleme aufwerfen kann und die Zusatzstoffe oftmals ebenfalls gesundheitlich nicht unbedenklich sind, werden Kühlschmiermittel ultravioletter Strahlung ausgesetzt, wodurch die Kühlschmiermittel sterilisiert werden sollen. In der Regel lässt sich durch Bestrahlung mit ultraviolettem Licht zwar keine völlige Keimfreiheit erzielen, es hat sich jedoch herausgestellt, dass es ausreichend ist, die Zahl der Keime unter einem gewissen Grenzwert zu halten, um die technische Funktion von Kühl- und/oder Schmiermitteln aufrechterhalten zu können.

Eine Vorrichtung zur Behandlung von derartigen Kühlschmiermitteln mit ultravioletter Strahlung ist im Stand der Technik beispielsweise aus der EP 1 005 954 B1 bekannt. Die darin vorgeschlagene Vorrichtung weist einen Gießkopf auf, über den die entsprechende Flüssigkeit eine drehbare Trägertrommel anströmt. Durch die Drehbewegung der Trägertrommel wird die angeströmte Flüssigkeit von der Trägertrommel mitgeführt, wodurch sich ein Flüssigkeitsfilm ausbildet, welcher von einer UV-Strahlungseinheit mit ultravioletter Strahlung bestrahlt wird. Zum Abtragen des Flüssigkeitsfilms ist der UV-Strahlungseinheit ein Abstreifer nachgeordnet. Nachteilig bei dieser Vorrichtung ist der konstruktiv relativ aufwendige Aufbau mit der drehbaren Trägertrommel. Weiterhin ist bei der Vorrichtung nachteilig, dass der konstruktive Aufbau der Vorrichtung Bereiche bedingt, an denen sich Flüssigkeit ansammeln kann, insbesondere im Bereich des Abstreifers und des Rahmengestells der Trägertrommel, ohne direkt einem Auffangbehälter zugeführt zu werden. Insofern können sich in diesen nicht abgeführten Flüssigkeitsansammlungen wiederum Keime bilden.

Vor diesem Hintergrund liegt der vorliegenden Erfindung die Aufgabe zugrunde, eine Vorrichtung zur Behandlung von flüssigen Medien bereitzustellen, die unter Meidung der oben genannten Nachteile konstruktiv einfacher und kostengünstiger herstellbar ist und dabei eine verhältnismäßig größere Bestrahlungsfläche aufweist.

Zur technischen Lösung der Aufgabe wird mit der vorliegenden Erfindung eine Vorrichtung gemäß Anspruch 1 vorgeschlagen.

Der Erfindung liegt die Erkenntnis zugrunde, dass durch im Wesentlichen spritzfreies Aufbringen eines flüssigen Mediums als Volumenstrom auf ein dreidimensionales Objekt, dessen Querschnittsfläche in einem Erzeugungsbereich in Fließrichtung eines aufgebrachten Mediums vergrößert ist, vorzugsweise im Wesentlichen gleichmäßig vergrößert ist, ein Medienfilm ausbildbar ist. An den Erzeugungsbereich schließt sich erfindungsgemäß ein Desinfektionsbereich an, welcher vorteilhafterweise eine im Wesentlichen konstante Querschnittsflächengröße aufweist. Während der ausgebildete Medienfilm sich in dem Desinfektionsbereich aufgrund der Gravitationskraft an dem Objekt in Richtung der Erdanziehungskraft bewegt, wird der Medienfilm erfindungsgemäß mit wenigstens einer ultravioletten Strahlungsquelle bestrahlt. Die wenigstens eine ultraviolette Strahlungsquelle ist dabei vorteilhafterweise fest oder beweglich um den Desinfektionsbereich beabstandet angeordnet, derart, dass der Medienfilm vorteilhafterweise gleichmäßig bestrahlbar ist. Bei der Bestrahlung des Medienfilms wird vorteilhafterweise zumindest ein Großteil von sich in dem flüssigen Medium befindlichen Keimen, wie Bakterien, Pilze und dergleichen Mikroorganismen, abgetötet. Vorzugsweise ist das dreidimensionale Objekt bezüglich seiner Längsachse im Wesentlichen in Richtung der Erdanziehungskraft ausgerichtet, wodurch ein homogener Medienfilm verbessert ausbildbar ist.

Eine besonders vorteilhafte Ausführungsform der vorliegenden Erfindung sieht vor, dass das dreidimensionale Objekt einen Abführbereich aufweist, in welchem die Querschnittsfläche des Objekts in Fließrichtung verringert ist, und in welchem sich unter Einwirkung der Gravitationskraft aufgrund der Geometrie des Abführbereichs aus dem Medienfilm ein Ablaufstrom ausbildet. Hierdurch ist vorteilhafterweise ein im Wesentlichen spritzfreies Abführen des flüssigen Mediums in die Auffangeinrichtung ermöglicht. Zudem verhindert der erfindungsgemäße Abführbereich vorteilhafterweise eine Ansammlung des flüssigen Mediums nach Durchlaufen des Desinfektionsbereiches an dem dreidimensionalen Objekt, wodurch vorteilhafterweise einer potenziellen Keimbildung in einer derartigen Flüssigkeits- beziehungsweise Feuchtigkeitsansammlung entgegengewirkt wird.

In einer weiteren vorteilhaften Ausführung der vorliegenden Erfindung weist das dreidimensionale Objekt einen im Wesentlichen elliptischen oder kreisförmigen Querschnitt auf. Vorteilhafterweise ist ein Objekt insbesondere mit einem kreisförmigen Querschnitt konstruktiv einfach herstellbar. Zudem ist durch einen elliptischen und/oder kreisförmigen Querschnitt ein homogener Medienfilm weiter verbessert ausbildbar.

Insbesondere zur weiteren Verbesserung der Ausbildbarkeit eines homogenen Medienfilms sieht eine vorteilhafte Ausführungsform der erfindungsgemäßen Vorrichtung vor, dass das dreidimensionale Objekt rotationssymmetrisch bezüglich seiner Längsachse ist.

In einer weiteren vorteilhaften Ausführungsform der Erfindung weist das dreidimensionale Objekt zumindest teilweise eine die ultraviolette Strahlung reflektierende Oberfläche auf. Vorteilhafterweise sind durch die reflektierten UV-Strahlen weitere Keime abtötbar. Vorzugsweise weist die UV-Strahlung zumindest näherungsweise eine Wellenlänge von 254 nm auf.

Eine weitere vorteilhafte Ausgestaltung der erfindungsgemäßen Vorrichtung sieht vor, dass das dreidimensionale Objekt eine Oberflächenstruktur zur Verbesserung der Fließeigenschaften des flüssigen Mediums aufweist. Durch die Oberflächenbeschaffenheit ist vorteilhafterweise insbesondere die Fließgeschwindigkeit des flüssigen Mediums beziehungsweise des Medienfilms beeinflussbar und somit verbessert ein ungewolltes Abreißen des Medienfilms verhinderbar. Zudem ist die Oberfläche derart gestaltbar, dass eine Ablagerung von in dem flüssigen Medium enthaltenen Schwebpartikel zumindest reduzierbar ist. Vorteilhafterweise ist das dreidimensionale Objekt frei von Senken, sodass vorteilhafterweise keine unerwünschten, eine Keimbildung begünstigende Ansammlungen von dem flüssigen Medium entstehen. Eine weitere vorteilhafte Ausgestaltung der Erfindung sieht darüber hinaus vor, dass der Druck des flüssigen Mediums in der Zuführung der Vorrichtung insbesondere zur Reinigung der Vorrichtung erhöhbar ist. Durch den erhöhten Druck und die damit verbundene erhöhte Fließgeschwindigkeit des flüssigen Mediums sind Verunreinigungen an dem dreidimensionalen Objekt vorteilhafterweise verbessert ablösbar und abschwemmbar. Zur Reinigung der Vorrichtung ist erfindungsgemäß vorgesehen, dass als flüssiges Medium Wasser und/oder eine Reinigungsflüssigkeit einbringbar ist, die über die Zuführung auf das dreidimensionale Objekt aufbringbar ist. Die Reinigungsflüssigkeit bewirkt dabei vorteilhafterweise ein verbessertes Ablösen von Ablagerungen und Verunreinigungen. Zudem ist erfindungsgemäß vorgesehen, dass insbesondere zur Reinigung des Objektes ein rohrartiger, vorzugsweise zylindrischer Hohlkörper im Wesentlichen zentrisch zumindest um das dreidimensionale Objekt anordbar ist, wobei das dreidimensionale Objekt und der Hohlkörper vorzugsweise gering beabstandet sind. Hierdurch ist vorteilhafterweise die Fließgeschwindigkeit des flüssigen Mediums weiter erhöhbar, wodurch die Reinigungswirkung weiter steigerbar ist. Des Weiteren stellt der Hohlkörper einen Spritzschutz dar, sodass insbesondere andere Anlagenteile und/oder Werkzeugmaschinen wirksam vor Feuchtigkeit und sich ablösenden Verunreinigungen geschützt sind.

In einer bevorzugten Ausführungsvariante der vorliegenden Erfindung entspricht das dreidimensionale Objekt in dem Erzeugungsbereich vorteilhafterweise im Wesentlichen einem Kegel oder einem Kegelstumpf. Es wurde festgestellt, dass sich die aufweitende Mantelfläche eines Kegels beziehungsweise eines Kegelstumpfes, insbesondere eines Kreiskegels, besonders vorteilhaft zur Ausbildung eines Medienfilms aus einem aufgebrachten Volumenstrom eignet.

Darüber hinaus ist eine Ausführungsvariante der Erfindung vorgesehen, in der das dreidimensionale Objekt in dem Abführbereich im Wesentlichen einem Kegel oder einem Kegelstumpf, vorzugsweise einem Kreiskegel, entspricht. Der Medienfilm ist bei einer derartigen geometrischen Gestaltung des Abführbereichs vorteilhafterweise gezielt in die Auffangeinrichtung abführbar, sodass diese vorteilhafterweise kleinflächig ausgestaltet ist.

In einer weiteren Ausführungsform der erfindungsgemäßen Vorrichtung ist das dreidimensionale Objekt vorteilhafterweise mit einer austauschbaren Oberflächenbespannung ausgestattet. Das dreidimensionale Objekt ist hierdurch kostengünstig als Rahmenkonstruktion ausgestaltbar, wobei durch die Oberflächenbespannung die erfindungsgemäße Oberfläche geschaffen wird. Vorteilhafterweise ist eine derartige Oberflächenbespannung einfach austauschbar, sodass bei verunreinigter Oberfläche eine aufwändige Reinigung beziehungsweise eine mit höheren Kosten verbundene Neubeschaffung eines neuen Objektes entbehrlich ist. Bei einer weiteren vorteilhaften Ausführungsform der erfindungsgemäßen Vorrichtung ist das dreidimensionale Objekt zur Ablösung von anhaftenden Verschmutzungen in Vibrationen versetzbar. Vorteilhafterweise sind ferner Mittel zur Filterung von organischen und/oder anorganischen Partikeln vorgesehen.

Vorteilhafterweise ist erfindungsgemäß ferner vorgesehen, dass zur Beeinflussung der Fließeigenschaften des flüssigen Mediums an der Oberfläche des dreidimensionalen Objekts eine elektrische Ladung einstellbar ist. Da der Medienfilm regelmäßig dünn ausgebildet ist, mitunter nur wenige zehntel Millimeter, ist der Medienfilm vorteilhafterweise durch eine elektrische Ladung in seinem Fließverhalten beeinflussbar. Auf diese Weise ist vorteilhafterweise steuernd ein homogener Medienfilm einstellbar.

Eine bevorzugte Ausgestaltung der vorliegenden Erfindung weist eine Einrichtung zur Bestimmung der Dicke des Medienfilms und eine Regeleinrichtung zur Regelung der Menge des zugeführten Medienstroms auf, wobei die Menge des zugeführten flüssigen Mediums in Abhängigkeit von der Dicke des Medienfilms regulierbar ist. Da insbesondere bei milchigen Flüssigkeiten die Eindringtiefe der ultravioletten Strahlen mitunter weniger als 1 mm beträgt, ist bei schwankender Dicke des Medienfilms das Ergebnis der Bestrahlung unterschiedlich. Die erfindungsgemäße Regeleinrichtung erlaubt dagegen vorteilhafterweise die Dicke des Medienfilms regelungstechnisch auf einem konstanten Niveau zu halten beziehungsweise zu überwachen, ob die Dicke des Medienfilmes konstant bleibt. Hierdurch ist vorteilhafterweise eine gleichbleibende Güte bei der Sterilisierung der Flüssigkeit erzielbar.

In einer vorteilhaften Ausgestaltung der erfindungsgemäßen Vorrichtung ist der Volumenstrom, welcher auf den Aufbringbereich des dreidimensionalen Objektes aufbringbar ist, im Wesentlichen turbulentfrei. Durch das Unterdrücken von Turbulenzen ist die Ausbildung eines homogenen Medienfilms im Erzeugungsbereich des dreidimensionalen Objektes vorteilhafterweise weiter verbessert. In einer weiteren besonders vorteilhaften Ausgestaltung der erfindungsgemäßen Vorrichtung weist die Zuführung für das zu behandelnde flüssige Medium Elemente zur Erzeugung eines im Wesentlichen laminaren Volumenstroms aufweist. Weiterhin ist vorteilhafterweise vorgesehen, dass der Volumenstrom einen im Wesentlichen kreisförmigen Querschnitt aufweist, wodurch die Ausbildung eines Medienfilms vorteilhafterweise weiter verbessert ist. Bei kreisförmiger Querschnittsfläche des Desinfektionsbereichs des dreidimensionalen Objektes entspricht die Querschnittsfläche des Volumenstroms dabei vorteilhafterweise zumindest näherungsweise der Querschnittsfläche des Desinfektionsbereiches multipliziert mit dem Verhältnis aus Solldicke des Medienfilms zu einem Viertel des Durchmessers des Querschnitts des Desinfektionsbereichs.

Als weitere vorteilhafte Ausführungsform der erfindungsgemäßen Vorrichtung wird vorgeschlagen, dass die Zuführung ein länglicher Hohlkörper ist, in dessen Volumenraum zumindest der Aufbringbereich des dreidimensionalen Objektes zumindest teilweise eingebracht ist, derart, dass die Austrittsöffnung der Zuführung verkleinert ist. Vorteilhafterweise ist die Zuführung nach Art eines Rohres ausgestaltet. In einer weiteren vorteilhaften Ausgestaltung ist die Zuführung in Verlängerung der Längsachse des dreidimensionalen Objekts angeordnet. Vorzugsweise umströmt das flüssige Medium den Bereich des in den Volumenraum der Zuführung eingebrachten dreidimensionalen Objektes derart, dass der aus der Zuführung austretende Volumenstrom sich im Wesentlichen turbulentfrei an den Erzeugungsbereich des dreidimensionalen Objektes anlegt und sich ein zumindest im Wesentlichen homogener Medienfilm mit zumindest näherungsweise konstanter Dicke ausbildet. Eine besonders vorteilhafte Ausgestaltung dieser Ausführungsform der erfindungsgemäßen Vorrichtung sieht eine Einstellbarkeit der Einbringtiefe des dreidimensionalen Objektes in den Volumenraum der Zuführung vor. Aufgrund der Geometrie des Erzeugungsbereichs des dreidimensionalen Objekts, dessen Querschnittsfläche in Fließrichtung vergrößert ist, ist vorteilhafterweise durch die Einstellbarkeit der Einbringtiefe die Größe der Austrittsöffnung der Zuführung veränderbar. Je weiter das dreidimensionale Objekt dabei in den Volumenbereich der Zuführung eingebracht wird, desto stärker verkleinert sich die Öffnung der Zuführung. Vorzugsweise sind die Zuführung und das dreidimensionale Objekt derart dimensioniert, dass ein Verschließen der Austrittsöffnung durch zumindest teilweises Einbringen des Erzeugungsbereiches in den Volumenraum der Zuführung ermöglicht ist. Besonders bevorzugt ist die Austrittsöffnung der Zuführung derart verkleinert, dass die Austrittsöffnung im Wesentlichen ein ringförmiger Spalt ist. Durch den ringförmigen Spalt wird das flüssige Medium vorteilhafterweise auf den Aufbringbereich des dreidimensionalen Mediums aufgebracht. In einer weiteren vorteilhaften Ausgestaltung der Vorrichtung sind der Aufbringbereich und der Erzeugungsbereich zumindest teilweise identisch.

Mit der vorliegenden Erfindung wird zur technischen Lösung der der Erfindung zugrunde liegenden Aufgabe des Weiteren ein Verfahren zur Behandlung von flüssigen Medien, insbesondere von Kühl- und Schmierstoffen, vorgeschlagen, wie in Anspruch 13 definiert.

Des Weiteren ist vorgesehen, dass bei dem Verfahren eine erfindungsgemäße Vorrichtung genutzt wird.

Weitere Einzelheiten, Merkmale und Vorteile der Erfindung werden nachfolgend anhand der in den Figuren der Zeichnung dargestellten Ausführungsbeispiele der Erfindung näher erläutert. Dabei zeigen:
- Fig. 1: in einer schematischen Seitenansicht eine Prinzipdarstellung einer Vorrichtung,
- Fig. 2: in einer schematischen Seitenansicht eine Prinzipdarstellung einer Vorrichtung,
- Fig. 3: in einer schematischen Seitenansicht eine Prinzipdarstellung einer Vorrichtung und
- Fig. 4: in einer schematischen Seitenansicht eine Prinzipdarstellung einer Ausführungsform einer erfindungsgemäßen Vorrichtung.

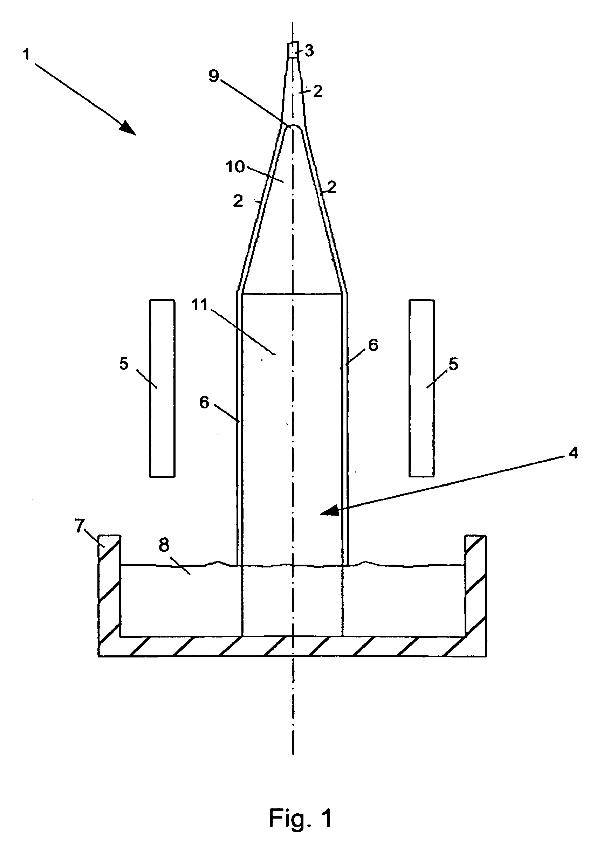

In Fig. 1 ist eine Vorrichtung 1 zur Sterilisierung von flüssigen Kühlschmiermitteln 2 dargestellt. Über eine als Rohrleitung ausgebildete Zuführung 3 sind Kühlschmiermittel 2, auf ein dreidimensionales Objekt 4 aufbringbar. In Fließrichtung des Kühlschmiermittels 2 weist das dreidimensionale Objekt 4 einen Aufbringbereich 9, einen Erzeugungsbereich 10 und einen Desinfektionsbereich 11 auf. Um den zylindrisch ausgebildeten Desinfektionsbereich 11 sind UV-Strahler 5 beweglich angeordnet. Die UV-Strahler sind dabei derart ausrichtbar, dass der Desinfektionsbereich 11 vollständig bestrahlbar ist, vorzugsweise mit einer Wellenlänge von 254 nm, die sich als äußerst wirksam zur Bekämpfung von Mikroorganismen erwiesen hat. In dem Ausführungsbeispiel wird das flüssige Kühlschmiermittel über die Rohrleitung 3 im wesentlichen spritz- und turbulentfrei auf den Aufbringbereich 9 des dreidimensionalen Objektes 4 aufgebracht. Durch die sich in Fließrichtung vergrößernde Querschnittsfläche des kreiskegelförmige Erzeugungsbereich 10 bildet sich unter Einwirkung der Gravitationskraft aus dem Kühlschmiermittel 2 ein Medienfilm 6 aus. Der ausgebildete Medienfilm 6 fließt in den Desinfektionsbereich 11, und bewegt sich in dem Desinfektionsbereich 11 unter

Einwirkung der Gravitationskraft in Richtung der Erdanziehungskraft, wobei der Medienfilm 6 von den UV-Strahlern 5 mit ultraviolettem Licht bestrahlt wird. Die Wellenlänge der ultravioletten Strahlung und die Dicke des Medienfilms 6 sind dabei derart aufeinander abgestimmt, dass der Medienfilm 6 im Wesentlichen vollständig von der ultravioletten Strahlung durchdrungen wird. Nach Durlaufen des Desinfektionsbereichs wird das bestrahlte Kühlschmiermittel 8 zur nachfolgenden Nutzung von einem Auffangbehälter aufgefangen. Alternativ kann das Kühlschmiermittel 8 direkt in eine Zuführung für eine Werkzeugmaschine weitergeleitet werden. Organische oder anorganische Partikel werden dabei vorteilhafterweise zuvor ausgefiltert, wozu die Vorrichtung geeignete Mittel aufweist, die in der Figur nicht explizit dargestellt sind.

Denkbar ist auch eine in den Figuren nicht dargestellte Ausgestaltung der erfindungsgemäßen Vorrichtung, bei der der Desinfektionsbereich zumindest im Übergangsbereich von Erzeugungsbereich auf den Desinfektionsbereich einen geringeren Durchmesser als der Erzeugungsbereich aufweist, sodass ein freifallender Medienfilm entsteht. Bevorzugt geht der Erzeugungsbereich aber stetig, wie beispielsweise in dem Ausführungsbeispiel in Fig. 1 dargestellt, in den Desinfektionsbereich über, sodass sich der Medienfilm an den Desinfektionsbereich anlegt.

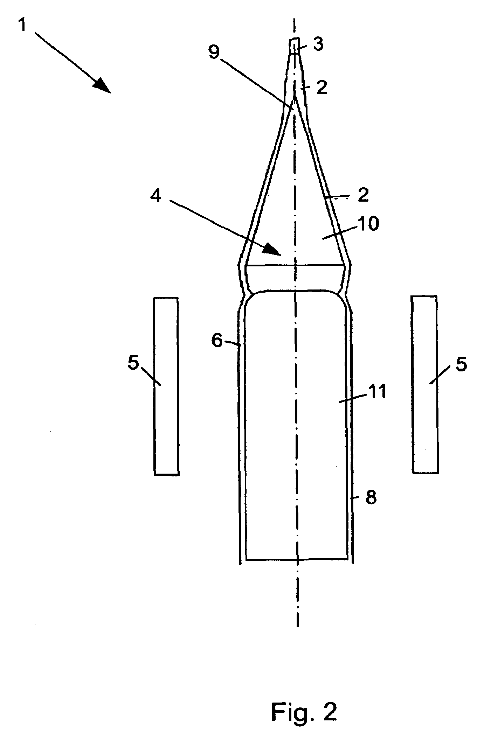

Fig. 2 zeigt eine Vorrichtung 1, wobei das rotationssymmetrische dreidimensionale Objekt 4 gegenüber dem Ausführungsbeispiel aus Fig. 1 eine geringfügig veränderte Geometrie aufweist. Im Übergang von dem Erzeugungsbereich 10, welcher im Wesentlichen einen Kreiskegel entspricht, auf den im Wesentlichen zylindrischen Desinfektionsbereich 11 weist das Objekt einen sich verjüngenden Bereich auf, durch den die Ausbildung des Medienfilms 6 vorteilhaft beeinflussbar ist. Darüber hinaus ist die Dicke des Medienfilms 6 mit Änderungen des Medienvolumenstroms 2 und/oder dessen Fließgeschwindigkeit an die spezifische Eindringtiefe der UV-Strahlen in den Medienfilm 6 anpassbar.

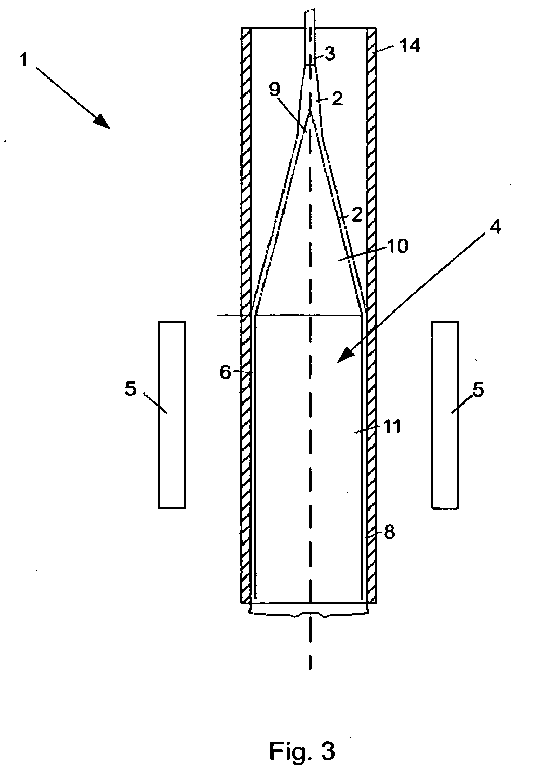

In Fig. 3 ist eine Vorrichtung 1 in einem Reinigungsmodus dargestellt. Die Vorrichtung 1 entspricht weitestgehend der in Fig. 1 dargestellten Vorrichtung, weist aber zusätzlich einen zylindrischen Hohlkörper 14 nach Art eines Rohres auf, welcher derart absenkbar ist, dass der Hohlkörper 14 zentrisch und gering beabstandet das dreidimensionale Objekt 4 umschließt. Das flüssige Medium 2, welches vorliegen mit Reinigungsmitteln versetztes Wasser ist, wird mit erhöhtem Druck aus der Zuführung 3 auf den Aufbringbereich 9 des dreidimensionalen Objektes 4 aufgebracht. Durch den erhöhten Druck und die erhöhte Fließgeschwindigkeit des flüssigen Mediums 2 werden Verunreinigungen und Ablagerungen (in der Figur nicht explizit dargestellt) von dem dreidimensionalen Objekt gelöst und abgeschwemmt. Der Hohlkörper 4 schützt dabei zudem die ultravioletten Strahlungsquellen 5 vor sich ablösenden Verunreinigungen und Feuchtigkeit.

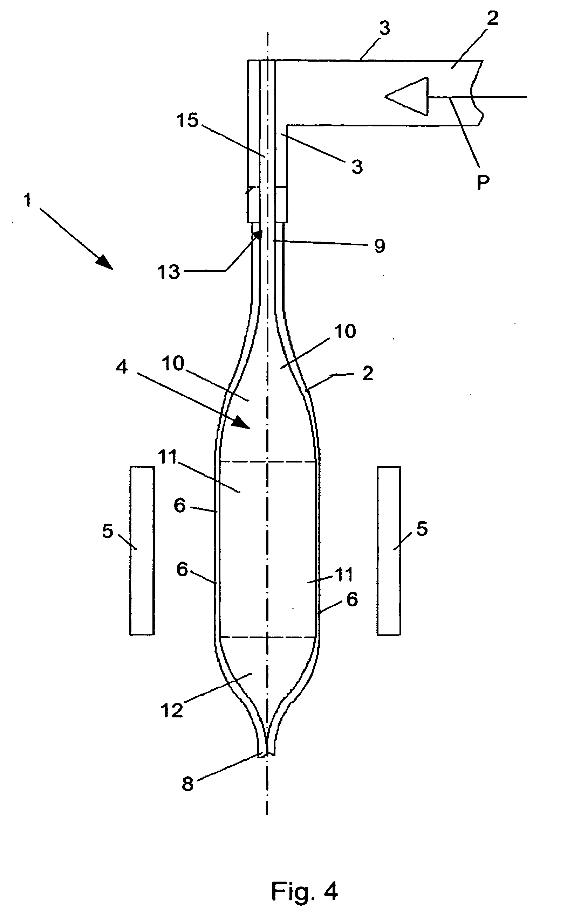

In Fig. 4 ist ein bevorzugtes Ausführungsbeispiel einer erfindungsgemäßen Vorrichtung dargestellt. Die Vorrichtung 1 weist ein rotationssymmetrisches dreidimensionales Objekt 4 auf, welches in seiner Längserstreckungsrichtung im Wesentlichen in Richtung der Erdanziehungskraft ausgerichtet ist, und auf welches ein flüssige Medium 2 aufbringbar ist. Das dreidimensionale Objekt 4 weist einen Aufbringbereich 9, einen Erzeugungsbereich 10, einen Desinfektionsbereich 11 und einen Abführbereich 12 auf. Die Zuführung 3 ist ein Rohrleitung, durch welche eine flüssiges Medium 2 in Pfeilrichtung P zugeführt wird. Vor der Austrittsöffnung 13 der Rohrleitung 3 weist die Rohrleitung 3 einen Winkel auf. In dem dem Winkel nachgeordneten Volumenraum der Rohrleitung 3 ist der im Wesentlichen zylindrische Aufbringbereich 9 des dreidimensionalen Objektes 4 zumindest teilweise eingebracht, wodurch sich die zylindrische Austrittsöffnung 13 der Rohrleitung 3 zu einem kreisförmigen Spalt verkleinert. Der Aufbringbereich 9 dient gleichsam als Aufhängung 15 des dreidimensionalen Objektes 4 in der Rohrleitung 3. Durch den in die Rohrleitung 3 zumindest teilweise eingebrachten Aufbringbereich 9, tritt das flüssige Medium 2 im Wesentlichen turbulent- und spritzfrei als ringförmiger Volumenstrom aus der Austrittsöffnung 13 der Rohrleitung 3 aus. Das dreidimensionale Objekt 4 ist durch ein Einschrauben weiter als in Fig. 4 dargestellt in den Volumenraum der Rohrleitung 3 einbringbar, wodurch die Größe des kreisförmigen Spaltes der Austrittsöffnung 13 vorteilhafterweise einstellbar ist und somit die Dicke des zu erzeugenden Medienfilms besser vorgebbar ist.

An den Aufbringbereich 9 schließt sich der Erzeugungsbereich 10 an, in welchem die Querschnittsfläche des Objektes 4 in Fließrichtung im Wesentlichen nach Art eines Kreiskegels vergrößert ist. Unter Einwirkung der Gravitationskraft bildet sich aufgrund der Geometrie des Erzeugungsbereichs 10 ein Medienfilm 6 aus. Der ausgebildete Medienfilm 6 fließt an dem zylindrischen Desinfektionsbereich 11 herab. Mehrere ultraviolette Strahlungsquellen 5 zur Bestrahlung des aus dem flüssigen Medium 2 erzeugten Medienfilms 6 sind radial um den Desinfektionsbereich angeordnet. Der Desinfektionsbereich 11 weist eine ultraviolette Strahlung reflektierende Oberfläche auf. Die Wellenlänge der ultravioletten Strahlung und die Dicke des Medienfilms 6 sind derart aufeinander abgestimmt, dass der Medienfilm 6 im Wesentlichen vollständig von der ultravioletten Strahlung durchdrungen wird.

An den Desinfektionsbereich 11 schließt sich ein Abführbereich 12 an, in welchem die Querschnittsfläche des dreidimensionalen Objekts 4 in Fließrichtung im Wesentlichen nach Art eines Kreiskegels verringert ist, und in welchem sich unter Einwirkung der Gravitationskraft aufgrund der Geometrie des Abführbereichs 12 aus dem Medienfilm 6 ein Ablaufstrom 8 ausbildet. Der Ablaufstrom 8 kann von einer in der Figur nicht explizit dargestellten Auffangeinrichtung für das behandelte flüssige Medium 8 zur nachfolgenden Nutzung des behandelten Mediums 8 aufgefangenen beziehungsweise direkt weitergeleitet werden.

### Bezugszeichenliste:

- 1: Vorrichtung
- 2: flüssiges Medium
- 3: Zuführung
- 4: dreidimensionales Objekt
- 5: ultraviolette Strahlungsquelle
- 6: Medienfilm
- 7: Auffangeinrichtung
- 8: behandeltes flüssiges Medium
- 9: Aufbringbereich
- 10: Erzeugungsbereich
- 11: Desinfektionsbereich
- 12: Abführbereich
- 13: Austrittsöffnung
- 14: Hohlkörper
- 15: Aufhängung
- P: Fluss des flüssigen Mediums

## Patentansprüche

1. Vorrichtung (1) zur Behandlung von flüssigen Medien (2), insbesondere von Kühl- und Schmierstoffen, aufweisend
wenigstens eine Zuführung (3) für das zu behandelnde flüssige Medium (2),
ein dreidimensionales Objekt (4), auf welches das flüssige Medium (2) aufbringbar ist,
wenigstens eine ultraviolette Strahlungsquelle (5) zur Bestrahlung eines aus dem flüssigen Medium (2) erzeugten Medienfilms (6),
wobei die Wellenlänge der ultravioletten Strahlung und die Dicke des Medienfilms (6) derart aufeinander abgestimmt sind, dass der Medienfilm (6) im Wesentlichen vollständig von der ultravioletten Strahlung durchdringbar ist,
und eine Auffangeinrichtung (7) für das behandelte flüssige Medium (8) zur nachfolgenden Nutzung des behandelten Mediums (8),
wobei
das dreidimensionale Objekt (4)
rotationssymmetrisch bezüglich seiner Längsachse ist
und
in Fließrichtung eines aufgebrachten flüssigen Mediums
einen Aufbringbereich (9),
auf welchen das flüssige Medium (2) als Volumenstrom spritzfrei aufbringbar ist,
einen Erzeugungsbereich (10),
in welchem die Querschnittsfläche des Objektes (4) in Fließrichtung vergrößert ist, und
in welchem sich unter Einwirkung der Gravitationskraft aufgrund der Geometrie des Erzeugungsbereichs (10) ein Medienfilm (6) ausbildet,
einen Desinfektionsbereich (11),
in welchem die Querschnittsfläche des Objektes (4) konstant ist, und
in welchem der Medienfilm (6) einer ultravioletten Bestrahlung durch die wenigstens eine ultraviolette Strahlungsquelle (5) aussetzbar ist und sich unter Einwirkung der Gravitationskraft in Richtung der Erdanziehungskraft bewegt,
**gekennzeichnet durch**
einen kegelförmigen Abführbereich (12),
in welchem die Querschnittsfläche des Objekts (4) in Fließrichtung verringert ist,
und
in welchem sich unter Einwirkung der Gravitationskraft aufgrund der Geometrie des Abführbereichs (12) aus dem Medienfilm (6) ein Ablaufstrom ausbildet,
wobei der Medienfilm (6) nach der Bestrahlung von der Auffangeinrichtung (7) auffangbar ist,
aufweist.

2. Vorrichtung (1) nach Anspruch 1, **dadurch gekennzeichnet, dass** das dreidimensionale Objekt (4) einen elliptischen Querschnitt oder einen kreisförmigen Querschnitt aufweist.

3. Vorrichtung (1) nach Anspruch 1 oder Anspruch 2, **dadurch gekennzeichnet, dass** das dreidimensionale Objekte (4) zumindest teilweise eine die ultraviolette Strahlung reflektierende Oberfläche aufweist.

4. Vorrichtung (1) nach einem der Ansprüche 1 bis 3, **dadurch gekennzeichnet, dass** das dreidimensionale Objekt (4) eine Oberflächenstruktur zur Verbesserung der Fließeigenschaften des flüssigen Mediums (2) aufweist.

5. Vorrichtung (1) nach einem der Ansprüche 1 bis 4, **dadurch gekennzeichnet, dass** das dreidimensionale Objekt (4) in dem Erzeugungsbereich (10) kegelförmig ist.

6. Vorrichtung (1) nach einem der Ansprüche 1 bis 5, **dadurch gekennzeichnet, dass** das dreidimensionale Objekt (4) mit einer austauschbaren Oberflächenbespannung ausgestattet ist.

7. Vorrichtung (1) nach einem der Ansprüche 1 bis 6, **dadurch gekennzeichnet, dass** das dreidimensionale Objekt (4) zur Ablösung von anhaftenden Verschmutzungen in Vibrationen versetzbar ist.

8. Vorrichtung (1) nach einem der Ansprüche 1 bis 7, **dadurch gekennzeichnet, dass** zur Beeinflussung der Fließeigenschaften des flüssigen Mediums (2) an der Oberfläche des dreidimensionalen Objekts (4) eine elektrische Ladung einstellbar ist.

9. Vorrichtung (1) nach einem der Ansprüche 1 bis 8 **gekennzeichnet durch** eine Einrichtung zur Bestimmung der Dicke des Medienfilms (6) und eine Regeleinrichtung zur Regelung der Menge des zugeführten Medienstroms, wobei die Menge des zugeführten flüssigen Mediums (2) in Abhängigkeit von der Dicke des Medienfilms (6) regulierbar ist.

10. Vorrichtung (1) nach einem der Ansprüche 1 bis 9, **dadurch gekennzeichnet, dass** der Volumenstrom turbulentfrei ist.

11. Vorrichtung (1) nach einem der Ansprüche 1 bis 10, **dadurch gekennzeichnet, dass** die Zuführung (3) für das zu behandelnde flüssige Medium (2) Elemente zur Erzeugung eines laminaren Volumenstroms aufweist.

12. Vorrichtung (1) nach einem der Ansprüche 1 bis 11, **dadurch gekennzeichnet, dass** der Volumenstrom einen kreisförmigen Querschnitt aufweist.

13. Verfahren zur Behandlung von flüssigen Medien (2), insbesondere von Kühl- und Schmierstoffen, wobei aus dem flüssigen Medium (2) nach Aufbringung auf ein dreidimensionales Objekt (4) ein Medienfilm (6) erzeugt wird, welcher unter Einwirkung von ultravioletter Strahlung sterilisiert wird,
**dadurch gekennzeichnet, dass**
das dreidimensionale Objekt (4)
rotationssymmetrisch bezüglich seiner Längsachse ist und
in Fließrichtung eines aufgebrachten flüssigen Mediums
einen Aufbringbereich (9),
auf welchen das flüssige Medium als Volumenstrom spritzfrei aufgebracht wird,
einen Erzeugungsbereich (10),
in welchem die Querschnittsfläche des Objektes (4) in Fließrichtung vergrößert ist, und
in welchem unter Einwirkung der Gravitationskraft aufgrund der Geometrie des Erzeugungsbereichs (10) ein Medienfilm (6) ausgebildet wird,
einen Desinfektionsbereich (11),
in welchem die Querschnittsfläche des Objektes (4) konstant ist, und
in welchem der Medienfilm (6) einer ultravioletten Bestrahlung durch wenigstens eine ultraviolette Strahlungsquelle (5) ausgesetzt wird und sich unter Einwirkung der Gravitationskraft in Richtung der Erdanziehungskraft bewegt,
und einen kegelförmigen Abführbereich (12)
in welchem die Querschnittsfläche des Objekts (4) in Fließrichtung verringert ist,
und
in welchem sich unter Einwirkung der Gravitationskraft aufgrund der Geometrie des Abführbereichs (12) aus dem Medienfilm (6) ein Ablaufstrom ausbildet,
wobei der Medienfilm (6) nach der Bestrahlung von einer Auffangeinrichtung (7) aufgefangen wird,
aufweist.

14. Verfahren nach Anspruch 13 **gekennzeichnet durch** Nutzung einer Vorrichtung (1) nach einem der Ansprüche 1 bis 12.

## Claims

1. A device (1) for treating liquid media (2), in particular cooling and lubricating agents, comprising
at least one supply (3) for the liquid medium (2) to be treated,
a three-dimensional object (4), onto which the liquid medium (2) can be applied,
at least one ultraviolet radiation source (5) for irradiating a medium film (6) produced from the liquid medium (2),
wherein the wave length of the ultraviolet radiation and the thickness of the medium film (6) are adapted to each other such that the medium film (6) can essentially be penetrated completely by the ultraviolet radiation,
and a catchment device (7) for the treated liquid medium (8) for the subsequent use of the treated medium (8),
wherein
the three-dimensional object (4)
is rotationally symmetric with respect to the longitudinal axis thereof
and
comprises,
in the flow direction of an applied liquid medium,
an application area (9),
onto which the liquid medium (2) can be applied as volume flow without splashing,
a production area (10),
in which the cross-sectional surface of the object (4) is enlarged in the flow direction, and
in which a medium film (6) is formed under the influence of the gravitational force due the geometry of the production area (10),
a disinfection area (11),
in which the cross-sectional surface of the object (4) is constant, and
in which the medium film (6) can be exposed to an ultraviolet irradiation by means of the at least one ultraviolet radiation source (5) and can move in the direction of gravity under the influence of the gravitational force, **characterized by**
a cone-shaped discharge area (12),
in which the cross-sectional surface of the object (4) is reduced in the flow direction,
and
in which an outlet flow is formed from the medium film (6) under the influence of the gravitational force due to the geometry of the discharge area (12), wherein the medium film (6) can be collected by the catchment device (7) after the irradiation.

2. A device (1) according to claim 1, **characterized in that** the three-dimensional object (4) comprises an elliptic cross section or a circular cross section.

3. A device (1) according to claim 1 or claim 2, **characterized in that** the three-dimensional object (4) at least partially comprises a surface reflecting the ultraviolet radiation.

4. A device (1) according to one of the claims 1 to 3, **characterized in that** the three-dimensional object (4) comprises a surface structure for improving the flow properties of the liquid medium (2).

5. A device (1) according to one of the claims 1 to 4, **characterized in that** the three-dimensional object (4) is cone-shaped in the production area (10).

6. A device (1) according to one of the claims 1 to 5, **characterized in that** the three-dimensional object (4) is provided with an exchangeable surface covering.

7. A device (1) according to one of the claims 1 to 6, **characterized in that** the three-dimensional object (4) can be caused to vibrate, in order to remove adhering dirt.

8. A device (1) according to one of the claims 1 to 7, **characterized in that** an electric charge can be set on the surface of the three-dimensional object (4) for influencing the flow properties of the liquid medium (2).

9. A device (1) according to one of the claims 1 to 8, **characterized by** a device for determining the thickness of the medium film (6) and a control device for controlling the amount of the supplied medium flow, wherein the amount of the supplied liquid medium (2) can be regulated in dependence on the thickness of the medium film (6).

10. A device (1) according to one of the claims 1 to 9, **characterized in that** the volume flow is free of turbulences.

11. A device (1) according to one of the claims 1 to 10, **characterized in that** the supply (3) of the liquid medium (2) to be treated comprises elements for creating a laminar volume flow.

12. A device (1) according to one of the claims 1 to 11, **characterized in that** the volume flow comprises a circular cross section.

13. A method for treating liquid media (2), in particular cooling and lubricating agents, wherein a medium film (6) is produced from the liquid medium (2) after application onto a three-dimensional object (4), which medium film will be sterilized under the influence of ultraviolet radiation,
**characterized in that**
the three-dimensional object (4)
is rotationally symmetric with respect to the longitudinal axis thereof
and
comprises,
in the flow direction of an applied liquid medium,
an application area (9),
onto which the liquid medium is applied as volume flow without splashing,
a production area (10),
in which the cross-sectional surface of the object (4) is enlarged in the flow direction, and
in which a medium film (6) is formed under the influence of the gravitational force due the geometry of the production area (10),
a disinfection area (11),
in which the cross-sectional surface of the object (4) is constant, and
in which the medium film (6) is exposed to an ultraviolet irradiation by means of at least one ultraviolet radiation source (5) and can move in the direction of gravity under the influence of the gravitational force,
and a cone-shaped discharge area (12),
in which the cross-sectional surface of the object (4) is reduced in the flow direction,
and
in which an outlet flow is formed from the medium film (6) under the influence of the gravitational force due to the geometry of the discharge area (12), wherein the medium film (6) is collected by a catchment device (7) after the irradiation.

14. A method according to claim 13, **characterized by** the use of a device (1) according to one of the claims 1 to 12.

## Revendications

1. Dispositif (1) de traitement de médias liquides (2), notamment de réfrigérants et de lubrifiants, comprenant
au moins une conduite d'amenée (3) du médium liquide (2) à traiter,
un objet tridimensionnel (4), sur lequel on peut appliquer le médium liquide (2),
au moins une source de rayonnement ultraviolet (5) destinée à irradier un film de médium (6) généré à partir du médium liquide (2),
la longueur d'onde du rayonnement ultraviolet et l'épaisseur du film de médium (6) étant adaptées l'une à l'autre de sorte que le film de médium (6) est essentiellement pénétrable de façon complète par le rayonnement ultraviolet,
et un dispositif de capture (7) du médium liquide traité (8) pour l'utilisation suivante du médium traité (8),
dans lequel
l'objet tridimensionnel (4)
est formé à symétrie de révolution par rapport à son axe longitudinal
et
il comprend, dans la direction d'écoulement d'un médium liquide appliqué,
une zone d'application (9),
sur laquelle on peut appliquer, sans éclaboussures, le médium liquide (2) comme un flux volumique,
une zone de génération (10),
dans laquelle la surface de section de l'objet (4) est élargie dans la direction d'écoulement, et
dans laquelle il se forme un film de médium (6) sous l'effet de la force de gravité à cause de la géométrie de la zone de génération (10),
une zone de désinfection (11),
dans laquelle la surface de section de l'objet (4) est constante, et
dans laquelle le film de médium (6) peut être soumis à une irradiation ultraviolette par moyen de l'au moins une source de rayonnement ultraviolet (5) et le film de médium se déplace dans la direction de la force d'attraction de la terre sous l'effet de la force de gravité,
**caractérisé par** une zone d'évacuation (12) conique,
dans laquelle la surface de section de l'objet (4) est réduite dans la direction d'écoulement,
et
dans laquelle un flux de sortie se forme à partir du film de médium (6) sous l'effet de la force de gravité à cause de la géométrie de la zone d'évacuation (12),
le film de médium (6) pouvant être collecté par le dispositif de capture (7) après l'irradiation.

2. Dispositif (1) selon la revendication 1, **caractérisé en ce que** l'objet tridimensionnel (4) comprend une section transversale elliptique ou une section transversale circulaire.

3. Dispositif (1) selon la revendication 1 ou la revendication 2, **caractérisé en ce que** l'objet tridimensionnel (4) comprend au moins partiellement une surface réfléchissant le rayonnement ultraviolet.

4. Dispositif (1) selon l'une des revendications 1 à 3, **caractérisé en ce que** l'objet tridimensionnel (4) comprend une structure de surface pour améliorer les propriétés d'écoulement du médium liquide (2).

5. Dispositif (1) selon l'une des revendications 1 à 4, **caractérisé en ce que** l'objet tridimensionnel (4) est conique dans la zone de génération (10).

6. Dispositif (1) selon l'une des revendications 1 à 5, **caractérisé en ce que** l'objet tridimensionnel (4) est muni d'une tension superficielle échangeable.

7. Dispositif (1) selon l'une des revendications 1 à 6, **caractérisé en ce que** l'objet tridimensionnel (4) peut être mis en vibration pour enlever des salissures adhérantes.

8. Dispositif (1) selon l'une des revendications 1 à 7, **caractérisé en ce qu'**on peut fixer une charge électrique sur la surface de l'objet tridimensionnel (4) pour influencer les propriétés d'écoulement du médium liquide (4).

9. Dispositif (1) selon l'une des revendications 1 à 8, **caractérisé par** un dispositif destiné à déterminer l'épaisseur du film de médium (6) et un dispositif de réglage destiné à régler la quantité du flux de médium amené, la quantité du médium liquide amené (2) étant réglable en fonction de l'épaisseur du film de médium (6).

10. Dispositif (1) selon l'une des revendications 1 à 9, **caractérisé en ce que** le flux volumique est exempt de turbulences.

11. Dispositif (1) selon l'une des revendications 1 à 10, **caractérisé en ce que** la conduite d'amenée (3) du médium liquide à traiter (2) comprend des éléments pour générer un flux volumique laminaire.

12. Dispositif (1) selon l'une des revendications 1 à 11, **caractérisé en ce que** le flux volumique comprend une section transversale circulaire.

13. Procédé de traitement de médias liquides (2), notamment de réfrigérants et de lubrifiants, dans lequel un film de médium (6) est généré à partir du médium liquide (2) après avoir appliqué celui-ci sur un objet tridimensionnel (4), lequel film de médium (6) est stérilisé sous l'effet d'un rayonnement ultraviolet,
**caractérisé en ce que**
l'objet tridimensionnel (4)
est formé à symétrie de révolution par rapport à son axe longitudinal et il comprend, dans la direction d'écoulement d'un médium liquide appliqué,
une zone d'application (9),
sur laquelle on applique, sans éclaboussures, le médium liquide (2) comme un flux volumique,
une zone de génération (10),
dans laquelle la surface de section de l'objet (4) est élargie dans la direction d'écoulement, et
dans laquelle il se forme un film de médium (6) sous l'effet de la force de gravité à cause de la géométrie de la zone de génération (10),
une zone de désinfection (11),
dans laquelle la surface de section de l'objet (4) est constante, et
dans laquelle le film de médium (6) est soumis à une irradiation ultraviolette par moyen d'au moins une source de rayonnement ultraviolet (5) et le film de médium se déplace dans la direction de la force d'attraction de la terre sous l'effet de la force de gravité,
et une zone d'évacuation (12) conique,
dans laquelle la surface de section de l'objet (4) est réduite dans la direction d'écoulement,
et
dans laquelle un flux de sortie se forme à partir du film de médium (6) sous l'effet de la force de gravité à cause de la géométrie de la zone d'évacuation (12),
le film de médium (6) étant collecté par un dispositif de capture (7) après l'irradiation.

14. Procédé selon la revendication 13, **caractérisé par** l'utilisation d'un dispositif (1) selon l'une des revendications 1 à 12.
